(19) Europäisches Patentamt
European Patent Office
Office européen des brevets

(11) Veröffentlichungsnummer: **0 037 927**
**A1**

(12) # EUROPÄISCHE PATENTANMELDUNG

(21) Anmeldenummer: **81102169.0**

(22) Anmeldetag: **23.03.81**

(51) Int. Cl.³: **C 07 D 413/12**, A 01 N 43/80 // C07D261/18

(30) Priorität: **11.04.80 DE 3013908**

(43) Veröffentlichungstag der Anmeldung: **21.10.81 Patentblatt 81/42**

(84) Benannte Vertragsstaaten: **AT BE CH DE FR GB IT LI LU NL SE**

(71) Anmelder: **BASF Aktiengesellschaft, Carl-Bosch-Strasse 38, D-6700 Ludwigshafen (DE)**

(72) Erfinder: **Ammermann, Eberhard, Dr., Sachsenstrasse 3, D-6700 Ludwigshafen (DE)**
Erfinder: **Theobald, Hans, Dr., Parkstrasse 2, D-6703 Limburgerhof (DE)**
Erfinder: **Zeeh, Bernd, Dr., Thorwaldsenstrasse 5, D-6700 Ludwigshafen (DE)**
Erfinder: **Pommer, Ernst-Heinrich, Dr., Berliner Platz 7, D-6703 Limburgerhof (DE)**

(54) **2-(N-Aryl-, N-isoxazolylcarbonyl)-aminobutyrolactone, Verfahren zu ihrer Herstellung und diese enthaltende Fungizide.**

(57) 2-(N-Aryl-, N-isoxazolylcarbonyl)-aminobutyrolactone der Formel

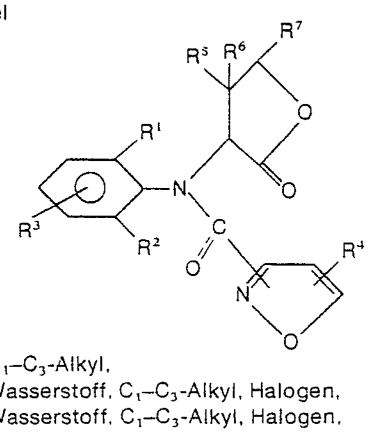

in der
$R^1$ $C_1$–$C_3$-Alkyl,
$R^2$ Wasserstoff, $C_1$–$C_3$-Alkyl, Halogen,
$R^3$ Wasserstoff, $C_1$–$C_3$-Alkyl, Halogen,
$R^4$ Wasserstoff, einen gegebenenfalls substituierten $C_1$–$C_3$-Alkylrest,
$R^5$, $R^6$ und $R^7$ Wasserstoff oder Methyl bedeuten und diese enthaltende Fungizide.

EP 0 037 927 A1

# 2-(N-Aryl-, N-isoxazolylcarbonyl)-aminobutyrolactone, Verfahren zu ihrer Herstellung und diese enthaltende Fungizide

Die vorliegende Erfindung betrifft neue 2-(N-Aryl-, N-isoxazolylcarbonyl)-aminobutyrolactone, Verfahren zu ihrer Herstellung, Fungizide, die diese Verbindungen als Wirkstoffe enthalten, sowie Verfahren zur Bekämpfung von Schadpilzen mit den Wirkstoffen.

2-(N-Aryl-, N-arylcarbonyl)-aminobutyrolactone, die fungizid wirksam sind, sind aus der US-PS 3 933 860 bekannt wie das 2-(N-2,6-Dimethylphenyl-, N-3,4-dichlorphenylcarbonyl)-aminobutyrolacton (Beispiel 2 der Patentschrift). Die Wirkung dieser Verbindungen gegen niedere Pilze wie Phytophthora ist jedoch unzureichend.

Es wurde nun gefunden, daß 2-(N-Aryl-, N-isoxazolylcarbonyl)-aminobutyrolactone der allgemeinen Formel I

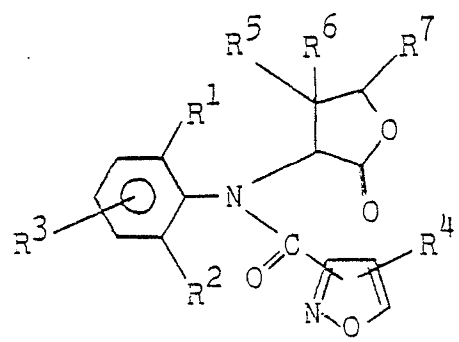

I

in der

$R^1$ $C_1$-$C_3$-Alkyl (Methyl, Äthyl),

$R^2$ Wasserstoff, $C_1$-$C_3$-Alkyl (Methyl, Äthyl), Halogen (Chlor, Brom),

$R^3$ Wasserstoff, $C_1$-$C_3$-Alkyl (Methyl, Äthyl), Halogen (Chlor, Brom),

$R^4$ Wasserstoff, einen gegebenenfalls durch Halogen (Chlor,

Sws/BL

BAD ORIGINAL

Brom) oder Alkoxy (Methoxy) substituierten $C_1$-$C_3$-Alkylrest (Methyl, Äthyl),

$R^5$, $R^6$ und $R^7$ unabhängig voneinander Wasserstoff oder Methyl bedeuten,

fungizid ausgezeichnet wirksam sind und den bekannten 2-(N-Aryl, N-arylcarbonyl)-aminobutyrolactonen in ihrer Wirkung, insbesondere gegen Phytophthora, überlegen sind.

Die 2-(N-Aryl-, N-isoxazolylcarbonyl)-aminobutyrolactone der Formel I besitzen in C-Atom 2 und gegebenenfalls in den C-Atomen 3 und 4 des Butyrolactonringes Asymmetriezentren.

Die optisch reinen Enantiomeren können nach üblichen Methoden erhalten werden. Fungizid wirksam sind sowohl die bei der Synthese üblicherweise anfallenden Gemische als auch die reinen Enantiomeren. Von der Erfindung werden sowohl die reinen Enantiomeren als auch ihre Gemische umfaßt.

Die 2-(N-Aryl-, N-isoxazolylcarbonyl)-aminobutyrolactone der Formel I können durch Umsetzung eines 2-(N-Aryl)-aminobutyrolactons der Formel II

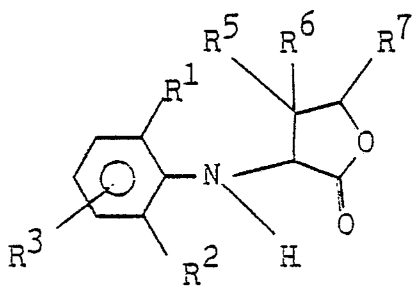

II

in der $R^1$, $R^2$, $R^3$, $R^5$, $R^6$ und $R^7$ die oben genannten Bedeutungen haben, mit einem Isoxazolylcarbonsäurederivat der Formel III

BAD ORIGINAL

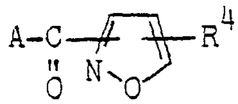

III

in der $R^4$ die oben genannte Bedeutung hat und A für eine nucleophil verdrängbare Abgangsgruppe steht, gegebenenfalls in Gegenwart eines Lösungs- oder Verdünnungsmittels, gegebenenfalls in Anwesenheit anorganischer oder organischer Basen und gegebenenfalls in Anwesenheit eines Reaktionsbeschleunigers bei einer Temperatur im Bereich zwischen 0 und 120°C erhalten werden.

In Formel III bedeutet A beispielsweise Halogen, wie Chlor oder Brom; Alkoxycarbonyloxyreste, wie Methoxycarbonyloxy oder Ethoxycarbonyloxy, oder den Benzyloxycarbonyloxyrest oder einen Azolylrest, wie den Imidazolyl- oder einen Triazolylrest.

Die Umsetzung kann in Gegenwart eines Lösungs- oder Verdünnungsmittels durchgeführt werden. Zu den bevorzugten Lösungs- bzw. Verdünnungsmitteln gehören Halogenkohlenwasserstoffe, beispielsweise Methylenchlorid, Chloroform, 1,2-Dichloräthan, Chlorbenzol; aliphatische oder aromatische Kohlenwasserstoffe, wie Cyclohexan, Petroläther, Benzol, Toluol oder Xylole; Ester wie Essigsäureäthylester; Nitrile, wie Acetonitril; Sulfoxide, wie Dimethylsulfoxid; Ketone, wie Aceton oder Methyläthylketon; Äther, wie Diäthyläther, Tetrahydrofuran oder Dioxan oder Gemische dieser Lösungsmittel.

Zweckmäßig verwendet man das Lösungs- bzw. Verdünnungsmittel in einer Menge von 100 bis 2000 Gew.%, vorzugsweise 100 bis 1000 Gew.%, bezogen auf die Ausgangsstoffe der Formel II bzw. III.

BAD ORIGINAL

Geeignete anorganische oder organische Basen, die gegebenenfalls als säurebindende Mittel dem Reaktionsgemisch zugesetzt werden können, sind beispielsweise Alkalicarbonate, wie Kalium- oder Natriumcarbonat; Alkalihydride, wie Natriumhydrid oder tertiäre Amine, wie Trimethylamin, Triäthylamin, N,N-Dimethylanilin, N,N-Dimethylcyclohexylamin, N-Methylpiperidin oder Pyridin; Azole, wie 1,2,4-Triazol oder Imidazol. Es können aber auch andere übliche Basen verwendet werden.

Als Reaktionsbeschleuniger kommen vorzugsweise Metallhalogenide, wie Natriumbromid oder Kaliumjodid, Azole, wie Imidazol oder 1,2,4-Triazol oder Pyridine, wie 4-Dimethylaminopyridin oder Mischungen dieser Substanzen in Betracht. Zweckmäßig setzt man auf 1 Mol Anilinderivat der Formel II 0,9 bis 1,3 Mol Säurederivat der Formel III sowie gegebenenfalls 0,5 bis 2 Mol Base und gegebenenfalls 0,01 bis 0,1 Mol Reaktionsbeschleuniger ein.

Die Umsetzung wird im allgemeinen bei einer Temperatur im Bereich zwischen 0 und 120°C in einem Zeitraum von 1 bis 60 Stunden, drucklos oder unter Druck, kontinuierlich oder diskontinuierlich durchgeführt.

In einer bevorzugten Form des erfindungsgemäßen Verfahrens vermischt man den Ausgangsstoff der Formel II gegebenenfalls mit einer Base und gegebenenfalls mit einem Verdünnungsmittel, gibt dann das Säurederivat der Formel III und gegebenenfalls den Reaktionsbeschleuniger zu und hält das Reaktionsgemisch für 0,5 bis 12, vorzugsweise 1 bis 6 Stunden bei der Reaktionstemperatur, die zwischen 0 und 120°C liegen kann.

Zur Isolierung der neuen Verbindungen wird beispielsweise, falls vorhanden, das Verdünnungsmittel entfernt, der

BAD ORIGINAL

Rückstand dann in einem geeigneten Lösungsmittel gelöst und die Lösung mit verdünnter Säure, dann mit wäßriger verdünnter Lauge sowie mit Wasser gewaschen, um die überschüssige Base und die Ausgangsstoffe II und III zu entfernen.

Die nach dem Abdestillieren des Lösungsmittels verbleibenden Produkte bedürfen im allgemeinen keiner weiteren Reinigung, können aber, falls erforderlich, nach bekannten Methoden, beispielsweise durch Umkristallisation, Extraktion oder Chromatographie, weiter gereinigt werden.

Die Aniline der Formel II sind bekannt oder können nach bekannten Verfahren hergestellt werden.

Die als Ausgangsstoffe eingesetzten Isoxazolylcarbonsäurederivate der Formel III sind teilweise bekannt bzw. können nach bekannten Methoden aus den Isoxazolylcarbonsäuren der Formel IV

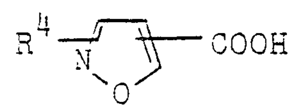 IV

in der $R^4$ die oben genannten Bedeutungen hat, hergestellt werden.

Die Carbonsäuren der Formel IV sind teilweise bekannt (Gazz. chim. ital. 72, 458-474 (1942), ibid 73, 764-768 (1948)) oder können durch Oxidation der bekannten Carbinole (Gazz. chim. ital. 69, 536-539 (1939)) hergestellt werden.

Am Beispiel der 3-Ethylisoxazolylcarbonsäure-(5) wird die Herstellung der Isoxazolylcarbonsäuren der Formel IV aus den entsprechenden Carbinolen durch Oxidation erläutert:

63,5 g 3-Ethyl-5-hydroxymethylisoxazol gelöst in 250 ml Diethylether werden bei 15 bis 20°C mit einer Mischung aus 750 ml Wasser, 164 g $Na_2Cr_2O_7 \cdot 2\ H_2O$ und 124 ml konzentrierter Schwefelsäure tropfenweise versetzt. Nach 12-stündigem Rühren bei 20 bis 25°C wird die organische Phase abgetrennt und über Natriumsulfat getrocknet. Nach Entfernung des Lösungsmittels erhält man 59 g 3-Ethylisoxazolylcarbonsäure-(5) vom Schmelzpunkt 194°C.

$C_6H_7NO_3$ (141)

| | | | |
|---|---|---|---|
| Ber.: | C 51,1 | H 5,0 | N 9,9 |
| Gef.: | C 51,9 | H 5,2 | N 10,7 |

Die folgende Tabelle gibt eine Übersicht über die Schmelzpunkte einiger Carbonsäuren der Formel IV:

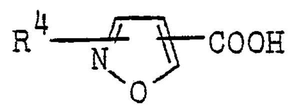 IV

Tabelle 1

| $R^4$ | Stellung der COOH-Gruppe am Isoxazolring | Fp. (°C) |
|---|---|---|
| H | 3 | 149 |
| H | 4 | 123 |
| H | 5 | 144 |
| 5-$CH_3$ | 3 | 176 |
| 3-$CH_3$ | 5 | 211 |

Einige Beispiele für die als Ausgangsstoffe eingesetzten Isoxazolylcarbonsäurederivate der Formel III (A = Cl) sind in der Tabelle 2 aufgeführt.

BAD ORIGINAL

Tabelle 2

| $R^4$ | Stellung der COCl-Gruppe am Isoxazolring | Kp ($^oC$/mbar) |
|---|---|---|
| H | 3 | 32-34/0,2 |
| H | 5 | 68-70/14 |
| 3-$CH_3$- | 5 | 40-45/0,25 |
| 3-$C_2H_5$- | 5 | 50-55/0,3 |
| 5-$ClCH_2$- | 3 | 79-80/0,05 |
| 5-$CH_3OCH_2$- | 3 | 72-73/0,2 |

Das folgende Beispiel erläutert die Herstellung der 2-(N-Aryl-, N-isoxazolylcarbonyl)-aminobutyrolactone der Formel I. Gewichtsteile verhalten sich zu Volumenteilen wie kg zu l.

Beispiel 1

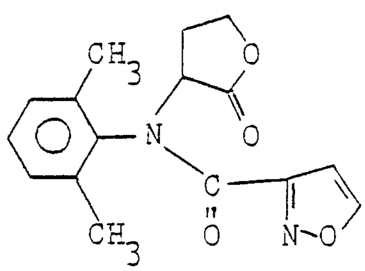

Bei 10°C werden zu einer Lösung von 16,4 g 2-(N-2,6-Dimethylphenyl)-aminobutyrolacton in 100 ml Methylenchlorid gleichzeitig aus zwei Tropftrichtern 8,5 g Triethylamin und 10,5 g Isoxazol-3-yl-carbonsäurechlorid zugetropft. Nach dem Rühren des Reaktionsgemisches über Nacht (12 Stunden) wird der Niederschlag abgesaugt und die organische Phase dreimal mit Wasser gewaschen, getrocknet und eingeengt. Aus dem verbleibenden Öl kristallisieren beim Verreiben in Diethylether 16 g 2-(N-2,6-Dimethylphenyl-N-

BAD ORIGINAL

-isoxazol-3-yl-carbonyl)-aminobutyrolacton vom Schmelzpunkt 112-114°C (Nr. 1).

$C_{16}H_{16}O_4N_2$ (300)

Ber.: C 64,0 H 5,3 N 9,3
Gef.: C 64,2 H 5,5 N 9,2

Analog lassen sich beispielsweise folgende Verbindungen der Formel I herstellen:

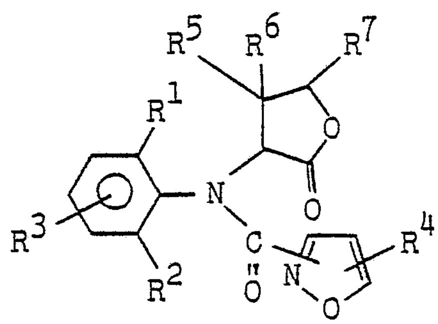

I

BAD ORIGINAL

Tabelle 3

| Nr. | $R^1$ | $R^2$ | $R^3$ | $R^4$ | $R^5$ | $R^6$ | $R^7$ | Position der CO-Gruppe am Isoxazolring | Phys. Daten |
|---|---|---|---|---|---|---|---|---|---|
| 2 | $CH_3$ | Cl | H | H | H | H | H | 3 | Fp. 100-103°C |
| 3 | $CH_3$ | $CH_3$ | H | H | H | H | H | 5 | Fp. 124-128°C |
| 4 | $CH_3$ | $CH_3$ | H | H | H | H | H | 4 | |
| 5 | $CH_3$ | $CH_3$ | 3-$CH_3$ | H | H | H | H | 3 | Fp. 132°C |
| 6 | $CH_3$ | $CH_3$ | 3-$CH_3$ | 5-$CH_2OCH_3$ | H | H | H | 3 | Öl |
| 7 | $CH_3$ | $CH_3$ | H | 5-$CH_2Cl$ | H | H | H | 3 | Fp. 101-105°C |
| 8 | $CH_3$ | $CH_3$ | H | 3-$CH_3$ | H | H | H | 5 | Fp. 162°C |
| 9 | $CH_3$ | $CH_3$ | 3-$CH_3$ | 3-$CH_3$ | H | H | H | 5 | Fp. 156-158°C |
| 10 | $CH_3$ | H | H | H | $CH_3$ | $CH_3$ | H | 3 | Öl |
| 11 | $CH_3$ | $CH_3$ | H | H | H | H | $CH_3$ | 3 | Fp. 130-134°C |
| 12 | $CH_3$ | $CH_3$ | 4-Br | H | H | H | H | 5 | |
| 13 | $CH_3$ | $CH_3$ | H | 5-$CH_2Cl$ | H | H | $CH_3$ | 3 | |
| 14 | $CH_3$ | Cl | H | 3-$CH_3$ | H | H | H | 5 | Fp. 156-158°C |
| 15 | $CH_3$ | H | H | H | H | H | H | 3 | Fp. 80°C |
| 16 | $CH_3$ | H | H | 3-$CH_3$ | H | H | H | 5 | Fp. 115°C |
| 17 | $C_2H_5$ | $CH_3$ | H | H | H | H | H | 3 | |
| 18 | $CH_3$ | $C_2H_5$ | H | 3-$CH_3$ | H | H | H | 5 | |

Die neuen Wirkstoffe weisen eine starke fungitoxische Wirkung auf. Sie sind geeignet zur Bekämpfung phytopathogener Pilze. Sie schädigen Kulturpflanzen in den zur Bekämpfung von Pilzen und Bakterien notwendigen Konzentrationen nicht. Aus diesen Gründen sind sie für den Gebrauch als Pflanzenschutzmittel zur Bekämpfung von Pilzen geeignet.

Sie sind beispielsweise geeignet zur Bekämpfung von Phytophthora cactorum an Äpfeln, Phytophthora infestans an Tomaten und Kartoffeln, Phytophthora parasitica an Erdbeeren, Pseudoperonospora cubensis an Gurken, Pseudoperonospora humuli an Hopfen, Peronospora destructor an Zwiebeln, Peronospora tabacina an Tabak, Peronospora sparsa an Rosen, Plasmopara viticola an Reben, Plasmopara halstedii an Sonnenblumen, Sclerospora macrospora an Mais, Bremia lactucae an Salat.

Die Aufwandmengen liegen je nach Art des gewünschten Effektes zwischen 0,025 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen. Darüber hinaus sind viele der neuen Verbindungen systemisch wirksam, so daß über die Wurzelbehandlung auch ein Schutz oberirdischer Pflanzen möglich ist.

Ferner lassen sich mit den neuen Verbindungen auch Pilze, die Keimlings- und Auflaufkrankheiten hervorrufen, beispielsweise Pythium- und Aphanomyces-Arten an Leguminosen und Baumwolle, bekämpfen. Die Aufwandmengen betragen je 100 kg Saatgut 10 bis 200 g Wirkstoff; die Anwendung erfolgt in Form von Saatgutbeizmitteln.

BAD ORIGINAL

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine oder Amide (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen - Fettalkohole - Ether, Alkylsulfonate und Arylsulfonate) und Dispergiermittel, wie Lignin, Sulfitablaugen und Methylcellulose.

Die fungiziden Mittel enthalten 0,1 bis 95 Gew.% Wirkstoff, vorzugsweise 0,5 bis 90 Gew.%.

BAD ORIGINAL

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile des Wirkstoffs Nr. 1 mit mit 10 Gewichtsteilen N-Methyl-α-pyrrolidon und erhält eine Lösung, die zur Anwendung in Form kleinster Tropfen geeignet ist.

II. 20 Gewichtsteile der Verbindung des Wirkstoffs Nr. 1 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.

III. 20 Gewichtsteile des Wirkstoffs Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

BAD ORIGINAL

IV. 20 Gewichtsteile des Wirkstoffs Nr. 2 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.

V. 20 Gewichtsteile des Wirkstoffs Nr. 5 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile des Wirkstoffs Nr. 3 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile des Wirkstoffs Nr. 6 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

BAD ORIGINAL

VIII. 40 Gewichtsteile des Wirkstoffs Nr. 2 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Ver- dünnen mit 100 000 Gewichtsteilen Wasser erhält man eine wäßrige Dispersion, die 0,04 Gew.-% Wirkstoff enthält.

IX. 20 Teile des Wirkstoffs Nr. 6 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und anderen Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Dithiocarbamate und deren Derivate, wie
Ferridimethyldithiocarbamat,
Zinkdimethyldithiocarbamat,

BAD ORIGINAL

Manganethylenbisdithiocarbamat,
Mangan-Zink-ethylendiamin-bis-dithiocarbamat,
Zinkethylenbisdithiocarbamat,
Tetramethylthiuramidsulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamt),
Ammoniak-Komplex von Zink-(N,N'-propylen-bis-dithiocarbamat) und
N,N'-Propylen-bis(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Substanzen, wie
N-Trichlormethylthio-tetrahydrophthalimid,
N-(1,1,2,2-Tetrachlorethylthio)-tetrahydrophthalimid,
N-Trichlormethylthio-phthalimid,
2-Heptadecyl-2-imidazolin-acetat,
2,4-Dichlor-6-(o-chloranilino)-s-triazin,
0,0-Diethyl-phthalimidophosphonothioat,
5-Amino-1-(bis-(dimethylamino)-phosphinyl)-3-phenyl-1,2,4--triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,

BAD ORIGINAL

2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-pyridinmethanol,
1,2-Bis-(3-ethoxycarbonyl-2-thioureido)-benzol,
1,2-Bis-(3-methoxycarbonyl-2-thioureido)-benzol

und verschiedene Fungizide, wie
Dodecylguanidinacetat,
3-(3-(3,5-Dimethyl-2-oxycyclohexyl)-2-hydroxyethyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-Benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,

5-Nitro-isophthalsäure-di-isopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(n-Propyl)-N-(2,4,6-trichlorphenoxyethyl)-N'-imidazolylharnstoff,
N-Cyclohexyl-N-methoxy-2,5-dimethyl-furan-3-carbonsäureamid,

BAD ORIGINAL

2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin.

Der folgende Versuch erläutert die fungizide Wirksamkeit der neuen Verbindungen.

Wirksamkeit gegen Phytophthora infestans an Tomaten

Blätter von Tomatenpflanzen der Sorte "Große Fleischtomate" werden mit wäßrigen Suspensionen, die 0,025, 0,006 und 0,003 % (Gewichtsprozent) Wirkstoff enthalten, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Bei dem Versuch zeigten die Wirkstoffe Nr. 1, 2, 3, 5, 6, 8, 9 eine bessere fungizide Wirksamkeit als der bekannte Wirkstoff entsprechend Beispiel 2 der US-PS 3 933 860.

BAD ORIGINAL

Patentansprüche

1. 2-(N-Aryl-, N-isoxazolylcarbonyl)-aminobutyrolactone der Formel I

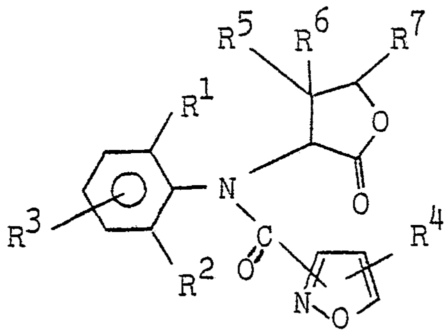

I

in der

$R^1$ $C_1$-$C_3$-Alkyl,
$R^2$ Wasserstoff, $C_1$-$C_3$-Alkyl, Halogen,
$R^3$ Wasserstoff, $C_1$-$C_3$-Alkyl, Halogen,
$R^4$ Wasserstoff, einen gegebenenfalls durch Halogen oder Alkoxy substituierten $C_1$-$C_3$-Alkylrest,
$R^5$, $R^6$ und $R^7$ unabhängig voneinander Wasserstoff oder Methyl bedeuten.

2. Fungizid enthaltend ein 2-(N-Aryl-, N-isoxazolylcarbonyl)-aminobutyrolacton der Formel I

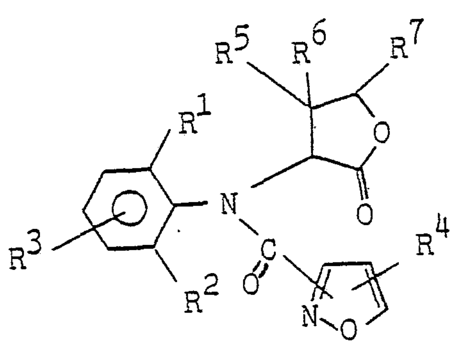

I

in der
$R^1$ $C_1$-$C_3$-Alkyl,
$R^2$ Wasserstoff, $C_1$-$C_3$-Alkyl, Halogen,
$R^3$ Wasserstoff, $C_1$-$C_3$-Alkyl, Halogen,
$R^4$ Wasserstoff, einen gegebenenfalls durch Halogen oder Alkoxy substituierten $C_1$-$C_3$-Alkylrest,
$R^5$, $R^6$ und $R^7$ unabhängig voneinander Wasserstoff oder Methyl bedeuten.

3. Fungizid, enthaltend einen festen oder flüssigen Trägerstoff und ein 2-(N-Aryl-, N-isoxazolylcarbonyl)-aminobutyrolacton der Formel

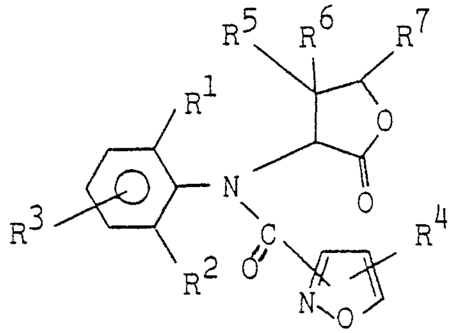

I

in der
$R^1$ $C_1$-$C_3$-Alkyl,
$R^2$ Wasserstoff, $C_1$-$C_3$-Alkyl, Halogen,
$R^3$ Wasserstoff, $C_1$-$C_3$-Alkyl, Halogen,
$R^4$ Wasserstoff, einen gegebenenfalls durch Halogen oder Alkoxy substituierten $C_1$-$C_3$-Alkylrest,
$R^5$, $R^6$ und $R^7$ unabhängig voneinander Wasserstoff oder Methyl bedeuten.

BAD ORIGINAL

4. Verfahren zur Herstellung eines Fungizids, dadurch gekennzeichnet, daß man einen festen oder flüssigen Trägerstoff vermischt mit einem 2-(N-Aryl-, N-isoxazolylcarbonyl)-aminobutyrolacton der Formel I

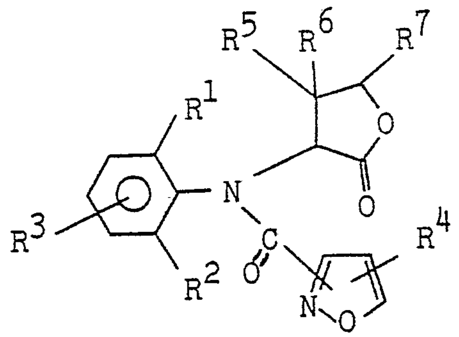

I

in der
$R^1$ $C_1$-$C_3$-Alkyl,
$R^2$ Wasserstoff, $C_1$-$C_3$-Alkyl, Halogen,
$R^3$ Wasserstoff, $C_1$-$C_3$-Alkyl, Halogen,
$R^4$ Wasserstoff, einen gegebenenfalls durch Halogen oder Alkoxy substituierten $C_1$-$C_3$-Alkylrest,
$R^5$, $R^6$ und $R^7$ unabhängig voneinander Wasserstoff oder Methyl bedeuten.

5. Verfahren zur Bekämpfung von Pilzen, dadurch gekennzeichnet, daß man die Pilze oder die vor Pilzbefall zu schützenden Gegenstände behandelt mit einem 2-(N-Aryl-, N-isoxazolylcarbonyl)-aminobutyrolacton der Formel I

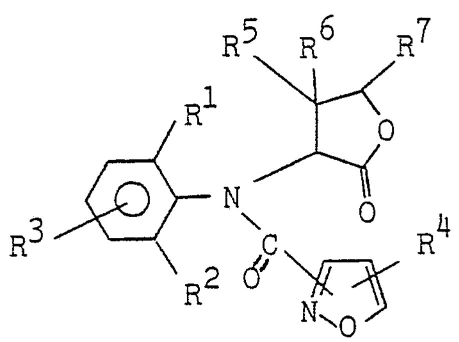

I

in der
$R^1$ $C_1$-$C_3$-Alkyl,
$R^2$ Wasserstoff, $C_1$-$C_3$-Alkyl, Halogen,
$R^3$ Wasserstoff, $C_1$-$C_3$-Alkyl, Halogen,
$R^4$ Wasserstoff, einen gegebenenfalls durch Halogen oder Alkoxy substituierten $C_1$-$C_3$-Alkylrest,
$R^5$, $R^6$ und $R^7$ unabhängig voneinander Wasserstoff oder Methyl bedeuten.

6. Verfahren zur Herstellung eines 2-(N-Aryl-, N-isoxazolylcarbonyl)-aminobutyrolactons der Formel I

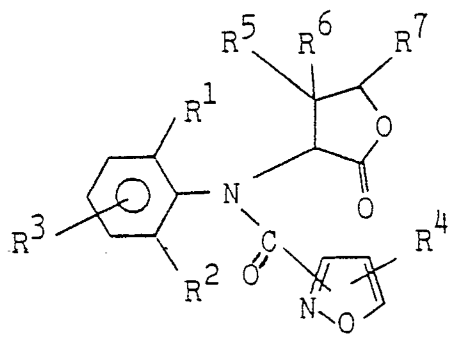

I

in der
$R^1$ $C_1$-$C_3$-Alkyl,
$R^2$ Wasserstoff, $C_1$-$C_3$-Alkyl, Halogen,
$R^3$ Wasserstoff, $C_1$-$C_3$-Alkyl, Halogen,
$R^4$ Wasserstoff, einen gegebenenfalls durch Halogen oder Alkoxy substituierten $C_1$-$C_3$-Alkylrest,
$R^5$, $R^6$ und $R^7$ unabhängig voneinander Wasserstoff oder Methyl bedeuten, dadurch gekennzeichnet, daß man ein 2-(N-Aryl)-aminobutyrolacton der Formel II

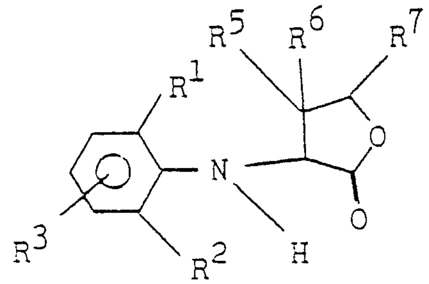

II

in der $R^1$, $R^2$, $R^3$, $R^5$, $R^6$ und $R^7$ die oben genannten Bedeutungen haben, mit einem Isoxazolylcarbonsäurederivat der Formel III

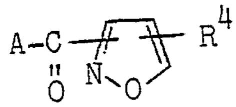

in der

$R^4$ die oben genannten Bedeutungen hat und

A für eine nucleophil verdrängbare Abgangsgruppe steht, gegebenenfalls in Gegenwart eines Lösungs- oder Verdünnungsmittels, gegebenenfalls in Anwesenheit anorganischer oder organischer Basen und gegebenenfalls in Anwesenheit eines Reaktionsbeschleunigers bei einer Temperatur im Bereich zwischen 0 und 120°C umsetzt.

7. 2-(N-Aryl-, N-isocazolylcarbonyl)-aminobutyrolactone ausgewählt aus der Gruppe bestehend aus 2-(N-2,6-Dimethylphenyl, N-isoxazol-3-ylcarbonyl)-aminobutyrolacton, 2-(N-2-Methyl-6-chlorphenyl)-, N-isoxazol-3-ylcarbonylU-aminobutyrolacton und 2-(N-2,6-Dimethylphenyl-, N-isoxazol-5-ylcarbonyl)-aminobutyrolacton.

8. Fungizid, enthaltend ein 2-(N-Aryl-, N-isoxazolylcarbonyl)-aminobutyrolacton ausgewählt aus der Gruppe bestehend aus 2-(N-2,6-Dimethylphenyl, N-isoxazol-3-ylcarbonyl)-aminobutyrolacton, 2-(N-2-Methyl-6-chlorphenyl-, N-isoxazol-3-ylcarbonyl)-aminobutyrolacton und 2-(N-2,6-Dimethylphenyl-, N-isoxazol-5-ylcarbonyl)-aminobutyrolacton.

BAD ORIGINAL

Patentansprüche (für Österreich)

1. Fungizid enthaltend ein 2-(N-Aryl-, N-isoxazolylcarbonyl)-aminobutyrolacton der Formel I

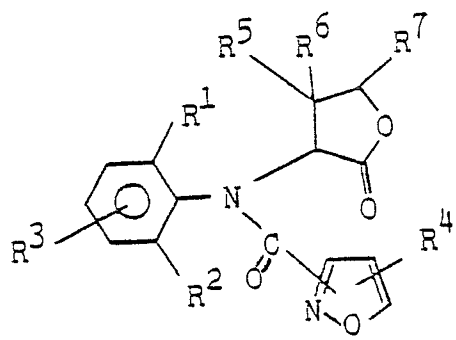

I

in der

$R^1$ $C_1$-$C_3$-Alkyl,

$R^2$ Wasserstoff, $C_1$-$C_3$-Alkyl, Halogen,

$R^3$ Wasserstoff, $C_1$-$C_3$-Alkyl, Halogen,

$R^4$ Wasserstoff, einen gegebenenfalls durch Halogen oder Alkoxy substituierten $C_1$-$C_3$-Alkylrest,

$R^5$, $R^6$ und $R^7$ unabhängig voneinander Wasserstoff oder Methyl bedeuten.

2. Fungizid, enthaltend einen festen oder flüssigen Trägerstoff und ein 2-(N-Aryl-, N-isoxazolylcarbonyl)-aminobutyrolacton der Formel

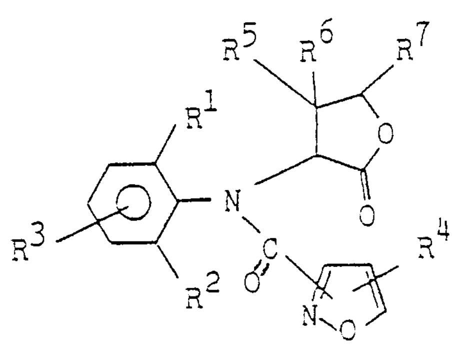

I

BAD ORIGINAL

in der
$R^1$ $C_1$-$C_3$-Alkyl,
$R^2$ Wasserstoff, $C_1$-$C_3$-Alkyl, Halogen,
$R^3$ Wasserstoff, $C_1$-$C_3$-Alkyl, Halogen,
$R^4$ Wasserstoff, einen gegebenenfalls durch Halogen oder Alkoxy substituierten $C_1$-$C_3$-Alkylrest,
$R^5$, $R^6$ und $R^7$ unabhängig voneinander Wasserstoff oder Methyl bedeuten.

3. Verfahren zur Herstellung eines Fungizids, dadurch gekennzeichnet, daß man einen festen oder flüssigen Trägerstoff vermischt mit einem 2-(N-Aryl-, N-isoxazolylcarbonyl)-aminobutyrolacton der Formel I

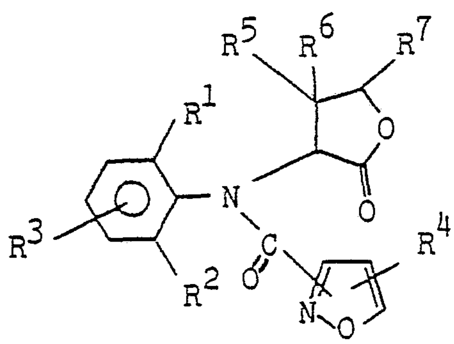

I

in der
$R^1$ $C_1$-$C_3$-Alkyl,
$R^2$ Wasserstoff, $C_1$-$C_3$-Alkyl, Halogen,
$R^3$ Wasserstoff, $C_1$-$C_3$-Alkyl, Halogen,
$R^4$ Wasserstoff, einen gegebenenfalls durch Halogen oder Alkoxy substituierten $C_1$-$C_3$-Alkylrest,
$R^5$, $R^6$ und $R^7$ unabhängig voneinander Wasserstoff oder Methyl bedeuten.

4. Verfahren zur Bekämpfung von Pilzen, dadurch gekennzeichnet, daß man die Pilze oder die vor Pilzbefall zu schützenden Gegenstände behandelt mit einem 2-(N-Aryl-, N-isoxazolylcarbonyl)-aminobutyrolacton der Formel I

BAD ORIGINAL

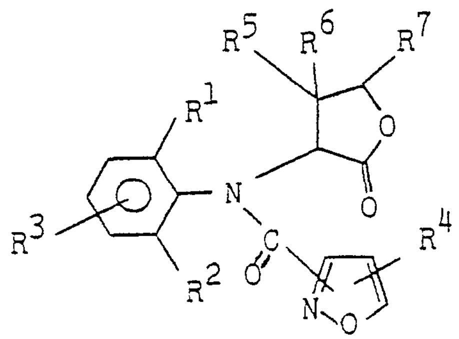

I

in der
$R^1$ $C_1$-$C_3$-Alkyl,
$R^2$ Wasserstoff, $C_1$-$C_3$-Alkyl, Halogen,
$R^3$ Wasserstoff, $C_1$-$C_3$-Alkyl, Halogen,
$R^4$ Wasserstoff, einen gegebenenfalls durch Halogen oder Alkoxy substituierten $C_1$-$C_3$-Alkylrest,
$R^5$, $R^6$ und $R^7$ unabhängig voneinander Wasserstoff oder Methyl bedeuten.

5. Verfahren zur Herstellung eines 2-(N-Aryl-, N-isoxazolylcarbonyl)-aminobutyrolactons der Formel I

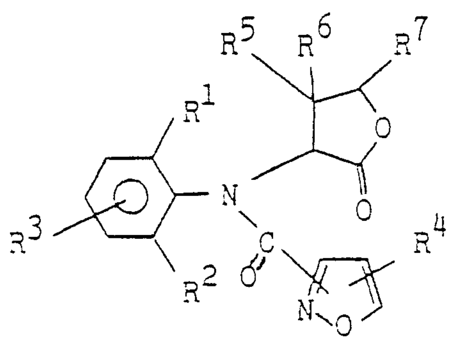

I

in der
$R^1$ $C_1$-$C_3$-Alkyl,
$R^2$ Wasserstoff, $C_1$-$C_3$-Alkyl, Halogen,
$R^3$ Wasserstoff, $C_1$-$C_3$-Alkyl, Halogen,
$R^4$ Wasserstoff, einen gegebenenfalls durch Halogen oder Alkoxy substituierten $C_1$-$C_3$-Alkylrest,

BAD ORIGINAL

$R^5$, $R^6$ und $R^7$ unabhängig voneinander Wasserstoff oder Methyl bedeuten, dadurch gekennzeichnet, daß man ein 2-(N-Aryl)-aminobutyrolacton der Formel II

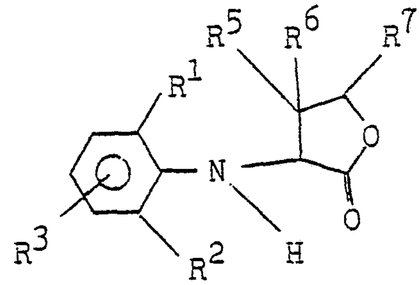

II

in der $R^1$, $R^2$, $R^3$, $R^5$, $R^6$ und $R^7$ die oben genannten Bedeutungen haben, mit einem Isoxazolylcarbonsäurederivat der Formel III

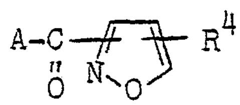

in der

$R^4$ die oben genannten Bedeutungen hat und

A für eine nucleophil verdrängbare Abgangsgruppe steht, gegebenenfalls in Gegenwart eines Lösungs- oder Verdünnungsmittels, gegebenenfalls in Anwesenheit anorganischer oder organischer Basen und gegebenenfalls in Anwesenheit eines Reaktionsbeschleunigers bei einer Temperatur im Bereich zwischen 0 und 120°C umsetzt.

6. Fungizid, enthaltend ein 2-(N-Aryl-, N-isoxazolylcarbonyl)-aminobutyrolacton ausgewählt aus der Gruppe bestehend aus 2-(N-2,6-Dimethylphenyl, N-isoxazol-3-ylcarbonyl)-aminobutyrolacton, 2-(N-2-Methyl-6-chlorphenyl-, N-isoxazol-3-ylcarbonyl)-aminobutyrolacton und 2-(N-2,6-Dimethylphenyl-, N-isoxazol-5-ylcarbonyl)-aminobutyrolacton.

BAD ORIGINAL

0037927

Europäisches Patentamt

# EUROPÄISCHER RECHERCHENBERICHT

Nummer der Anmeldung
EP 81 10 2169

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 513 732 (CIBA-GEIGY) * Ansprüche 1,19,21 * -- | 1-5 |
| P | US - A - 3 933 860 (DAVID CHEONG KMY CHAN) * Anspruch 1; Spalten 7,8 * -- | 1-5 |
| P | EP - A - 0 026 873 (BASF AG) * Ansprüche * ---- | 1-5 |

KLASSIFIKATION DER ANMELDUNG (Int. Cl.)

C 07 D 413/12
A 01 N 43/80//
C 07 D 261/18

RECHERCHIERTE SACHGEBIETE (Int. Cl.)

C 07 D 413/12
A 01 N 43/80

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 07-07-1981 | HENRY |